Europäisches Patentamt

**European Patent Office**

Office européen des brevets

(19)

(11) Publication number: **0 178 744**
A2

(12) **EUROPEAN PATENT APPLICATION**

(21) Application number: **85302186.3**

(51) Int. Cl.⁴: **C 12 N 15/00**

(22) Date of filing: **28.03.85**

(30) Priority: **12.10.84 GB 8425778**

(43) Date of publication of application:
**23.04.86 Bulletin 86/17**

(84) Designated Contracting States:
**AT BE CH DE FR GB IT LI NL SE**

(71) Applicant: **Antibioticos, S.A.**
**Calle Bravo Murillo, no. 38**
**28003 Madrid(ES)**

(72) Inventor: **Sanchez, Flora**
**c/o Antibioticos, S.A. Bravo Murillo, 38**
**E-28015, Madrid(ES)**

(72) Inventor: **Garcia, Jose Luis**
**c/o Antibioticos, S.A. Bravo Murillo, 38**
**E-28015, Madrid(ES)**

(72) Inventor: **Penalva, Miguel A.**
**c/o Antibioticos, S.A. Bravo Murillo, 38**
**E-28015, Madrid(ES)**

(72) Inventor: **Marquez, Gabriel**
**c/o Antibioticos, S.A. Bravo Murillo, 38**
**E-28015, Madrid(ES)**

(72) Inventor: **Tourino, Angeles**
**c/o Antibioticos, S.A. Bravo Murillo, 38**
**E-28015, Madrid(ES)**

(72) Inventor: **Rubio, Victor**
**c/o Antibioticos, S.A. Bravo Murillo, 38**
**E-28015, Madrid(ES)**

(74) Representative: **Ruffles, Graham Keith et al,**
**MARKS & CLERK 57-60 Lincoln's Inn Fields**
**London WC2A 3LS(GB)**

(54) **Shuttle vector.**

(57) A plasmid such as the plasmid pFJ1 shown in the drawing and contained in *Bacillus subtilis* NCIB 12020 or *Brevibacterium lactofermentum* NCIB 12021 or *Escherichia coli* NCIB 12023 is capable of autonomous replication in microorganisms belonging to the genus *Corynebacterium* or the genus *Brevibacterium* and also in the species *Bacillus subtilis* and *Escherichia coli*.

EP 0 178 744 A2

Croydon Printing Company Ltd.

M&C FOLIO: 230P49007          WANGDOC: 0433C

## SHUTTLE VECTOR

The present invention relates to novel plasmids capable of autonomous replication in microorganisms belonging to different genera. Such multiply replicable plasmids are known as shuttle vectors.

Recombinant DNA techniques allow the controlled introduction of genes in to a microbial system and their expression, which may result in useful overproduction of some metabolites. More specifically, a vector (for example, a plasmid or a bacteriophage) is an important requirement for introducing controlled genetic information in to a microorganism using genetic engineering.

The present invention particularly relates to vectors for use in coryneform bacteria (that is, bacteria of the genera Brevibacterium and Corynebacterium). Coryneform bacteria are the main industrial producers of amino acids. The development of a vector able to replicate in the coryneform system allows the cloning of a gene capable of directing the synthesis of enzymes related to the biosynthesis of amino acids.

GB2076853 is concerned with a coryneform bacterium for production of L-glutamic acid. A DNA fragment from an L-glutamic acid-producing coryneform bacterium is used to generate a hybrid plasmid for insertion in to a coryneform bacterium.

FR2482622 describes production of L-lysine using an engineered coryneform bacterium in which a DNA is inserted which controls resistance to S-(2-aminoethyl)cysteine and production of L-lysine.

EP63764 discloses transformation of microorganisms belonging to the genera Corynebacterium and Brevibacterium by incubating protoplasts of a microbial strain belonging to one of the genera and a donor deoxyribonucleic acid in the presence polyethyleneglycol or polyvinylalcohol and at least one divalent metal cation to introduce said deoxyribonucleic acid into the protoplasts, regenerating the protoplasts by culturing in a hypertonic nutrient medium and thereafter recovering a transformed strain having a phenotype derived from the donor deoxyribonucleic acid.

EP66129 is directed to the production of L-threonine using an engineered Corynebacteria or Brevibacteria in which a fragment of chromosomal DNA derived from a DNA-donor resistant to α-amino-β-hydroxyvaleric acid is inserted.

EP71023 is similarly concerned with the production of L-isoleucine using an engineered coryneform organism in which has been inserted a fragment of chromosomal DNA derived from a DNA-donor resistant to α-amino-β-hydroxyvaleric acid.

EP77548 is concerned with plasmids from Corynebacteria or Brevibacteria, particularly plasmid pAM330 obtained from ATCC 13869 and pAM286 from FERM-P5485. Such plasmids are also disclosed in some of the earlier specifications mentioned above. EP77548 describes insertion of coryneform chromosomal DNA in to the plasmids to enhance production of L-glutamic acid.

EP82485 is principally concerned with recombinant DNA vector plasmids which express a product in Corynebacteria or Brevibacteria. The plasmid is a recombinant of a DNA fragment containing a gene expressible in a microorganism belonging to the genus Corynebacterium or Brevibacterium and a plasmid autonomously replicable in a microorganism of the genus Corynebacterium or Brevibacterium. The recombinant is autonomously replicable in a microorganism of the genus Corynebacterium or Brevibacterium and is detectable by the expression of the introduced gene.

EP88166 is generally directed to cultivation methods based on insertion of a a gene in to the DNA of <u>Corynebacteria</u> or <u>Brevibacteria</u>. A host microorganism selected from microorganisms belonging to the genus <u>Corynebacterium</u> or <u>Brevibacterium</u> is transformed with a recombinant DNA containing DNA foreign to the host microorganism.

In general, the above patent literature suggests the possibility of genetically engineering coryneform bacteria to modify their industrially useful attributes. The DNA of these engineered bacteria is however principally obtained from <u>Corynebacteria</u> or <u>Brevibacteria</u>, and the resultant bacteria themselves are <u>Corynebacteria</u> or <u>Brevibacteria</u>. Hence development work is confined to these species.

In accordance with this invention there is provided a plasmid capable of autonomous replication in microorganisms belonging to the genus <u>Corynebacterium</u> or the genus <u>Brevibacterium</u> and also in of the species <u>Bacillus</u> <u>subtilis</u> and <u>Escherichia</u> <u>coli</u>.

The provision of a plasmid vector which can autonomously replicate in <u>Bacillus</u> <u>subtilis</u> and <u>Escherichia</u> <u>coli</u> allows study in these familiar species as well as use in coryneform bacteria.

The plasmid can usefully contain a selectable marker such as antibiotic resistance, as well as an origin of replication for coryneform bacteria, _Bacillus_ _subtilis_ and _Escherichia_ _coli_. The plasmids of this invention can also usefully include at least one uniquely cleavable restriction site for introduction of foreign DNA sequences. Such sites then allow engineering of developed forms in which the vector further contains a DNA sequence relevant to the production of a metabolite such as an amino acid.

The shuttle vectors of this invention can readily be constructed from available DNA sequences imparting the desired functions. Such available sequences can be plasmidal or chromosomal DNA. The typical plasmid of this invention, designated pFJ1, has been constructed from three plasmid sources by conventional gene-splicing techniques.

The typical plasmid pFJ1 is able to replicate and express a marker, tetracycline resistance, in coryneform bacteria, being a useful vector for them. It is also functional in _Escherichia_ _coli_ and _Bacillus_ _subtilis_ which allows the study of the genes inserted in the vector in the two better known systems of gram negative and gram positive bacteria, respectively.

This typical vector has been obtained by inserting a natural cryptic plasmid from Brevibacterium lactofermentum (pBL1) into a vector of Escherichia coli (pBR325) to give a hybrid molecule in to which has been added a Bacillus subtilis vector (pBC16.1) carrying a tetracycline resistance marker. The resulting molecule pFJ1 is able to transform and express tetracycline resistance in the three systems.

The plasmid, pBL1 (4.4 kilobases) was found in the strain Brevibacterium lactofermentum ATCC 13869, and is probably the same as plasmid pAM330 described in GB2076853. The plasmid pBL1 has the disadvantage of not conferring any detectable phenotypic characteristic to the strain. Another larger plasmid pBL2 was also found in the ATCC 13869 strain.

A restriction map for the plasmid pBL1 is included in the accompanying drawing, in which:
The Figure is a scheme for construction of the plasmid pFJ1.

Plasmid pBR325 is an Escherichia coli vector described by F.Bolivar in Gene 4 121 (1978)). It has tetracycline resistance. Like pBL1, the plasmid pBR325 has a single site for the restriction enzyme Hind III. Thus cleavage of both plasmids with Hind III and subsequent ligation readily gives a hybrid plasmid designated pFL1.

Insertion at the Hind III site of pBR325 inactivates its tetracycline resistance and hence clones containing hybrid molecules were easily detected. Plasmid pFL1 was found to be able to transform protoplasts of coryneform bacteria expressing chloramphenicol resistance.

To make this plasmid pFL1 functional in Bacillus subtilis, plasmid pBC16.1 (Kreft, J. et al. Molec. Gen. Genet. 162 59 (1978) and Tanaka, T. et al. J. Bacteriol. 129 1487 (1977)) can be employed. This plasmid is capable of conferring tetracycline resistance. Like plasmid pFL1, the plasmid pBC16.6 has a single site for the restriction enzyme EcoR I. Thus cleavage of both plasmids with EcoR I and subsequent ligation readily gives the plasmid designated pFJ1. The resulting hybrid molecule was obtained by transformation of Escherichia coli competent cells, selecting for tetracycline resistance. This drug marker is expressed in Escherichia coli (Kreft, J. et al. Molec. gen. Genet. 162 59 (1978).

The plasmid pFJ1 can then be introduced in to Bacillus subtilis and in Brevibacterium or Corynebacterium strains by conventional procedures, for example by treatment of protoplasts with polyethylene glycol (PEG) and selecting for transformants in regeneration media containing tetracycline.

Plasmid pFJl is able to express ampicillin resistance in Escherichia coli and tetracycline resistance in Escherichia coli, Bacillus subtilis and Brevibacterium or Corynebacterium strains. Strains of Bacillus subtilis, Brevibacterium lactofermentum ATCC 13869, and Escherichia coli K12 DH1 carrying pFJl have been deposited at the National Collection of Industrial Bacteria under repective accession numbers NCIB 12020 (3 october 1984), 12021 (3 october 1984) and 12023 (19 october 1984). The present invention is not however limited to the particular plasmid pFJl. Plasmids showing substantial homology (preferably >70%, more preferably >80% homology) with pFJl are easily constructed. More generally, alternative functionally equivalent DNA sequences can be substituted for those mentioned above.

The present invention is illustrated by the following example when taken in conjunction with the accompanying drawing.

E X A M P L E

(a) Purification of plasmid pBLl from Brevibacterium lactofermentum ATCC 13869.

The publically available strain ATCC 13869 was used to inoculate a semisynthetic medium (composition per liter:

glucose 20g, $(NH_4)_2SO_4$ 10g, urea 3g, yeast extract 1g, $KH_2PO_4$ 1g, $MgSO_4$ 0.4mg, NaCl 50mg, $FeSO_4$ 2µg, $MnSO_4$ 2 µg, biotin 50 µg and thiamine 200 µg, with medium pH adjusted to 7.3), and grown at 30°C for 20 hours. 500 ml of the same medium were inoculated with 5 ml of the previous culture and incubated at 30°C with shaking until the midlogaritmic phase of growth was reached. At this point Penicillin G was added at a final concentration of 0.3 U/ml and incubation was continued at 30°C for 90 minutes.

Cells were recovered by centrifugation and washed with TE buffer (Tris-hydroxymethylaminomethane 10mM pH 8.0, EDTA 1mM) and frozen. Thawed cells were resuspended in 40ml of solution I (glucose 50mM, Tris.HCl 25mM pH 8.0, EDTA 10mM pH 8.0, lysozyme 10mg/ml) and incubated at 37°C for 60 minutes. Then 80 ml of solution II (SDS 1%, NaOH 0.2M) were added and the mixture was incubated 5 minutes at 4°C; cells lysed at this point. Then 40 ml of 3M potassium acetate pH 4.8 were added and the lysate was maintained at 4°C for 60 minutes. Centrifugation was carried out at 8000g for 30 minutes at 4°C. To the supernatant 0.6 volumes of isopropanol were added, mixed and let stand at room temperature for 30 minutes. Then the mixture was centrifuged at 8000g during 5 minutes at room temperature. The supernatant was discarded and the pellet dried. The pellet was dissolved in 4.5 ml of TE buffer and the pH was adjusted to 7.0 with Tris-base;

the final volume was adjusted to 8 ml. This solution was mixed with 8g of CsCl, 1 ml of 5 mg/ml ethidium bromide and centrifuged at 40000 rpm for 40 hours at 25°C in a Ti50 rotor.

After density gradient centrifugation, bands of covalent closed circular DNA (lower) and linear DNA (upper) were distinguished by illumination with UV light. The lower band was collected from the side through a 21 gauge hypodermic needle with a syringe. Ethidium bromide was removed by extraction with isopropanol saturated with cesium chloride, and cesium chloride was eliminated by dialysis against TE buffer. In this way the plasmid DNA was obtained.

The plasmid DNA was subjected to agarose gel electrophoresis, showing a plasmid designated pBL1 with a molecular weight of 4.4 Kb assessed by comparing its electrophoresis mobility with that of appropriate standard covalent closed circular DNA size markers. Another plasmid pBL2 of MW 60Kb was also found, but it was not used in these studies. Plasmid pBL1 was separated from pBL2 by low molecular weight agarose gel electrophoresis.

(b) Cleavage sites of pBL1 for several restriction endonucleases

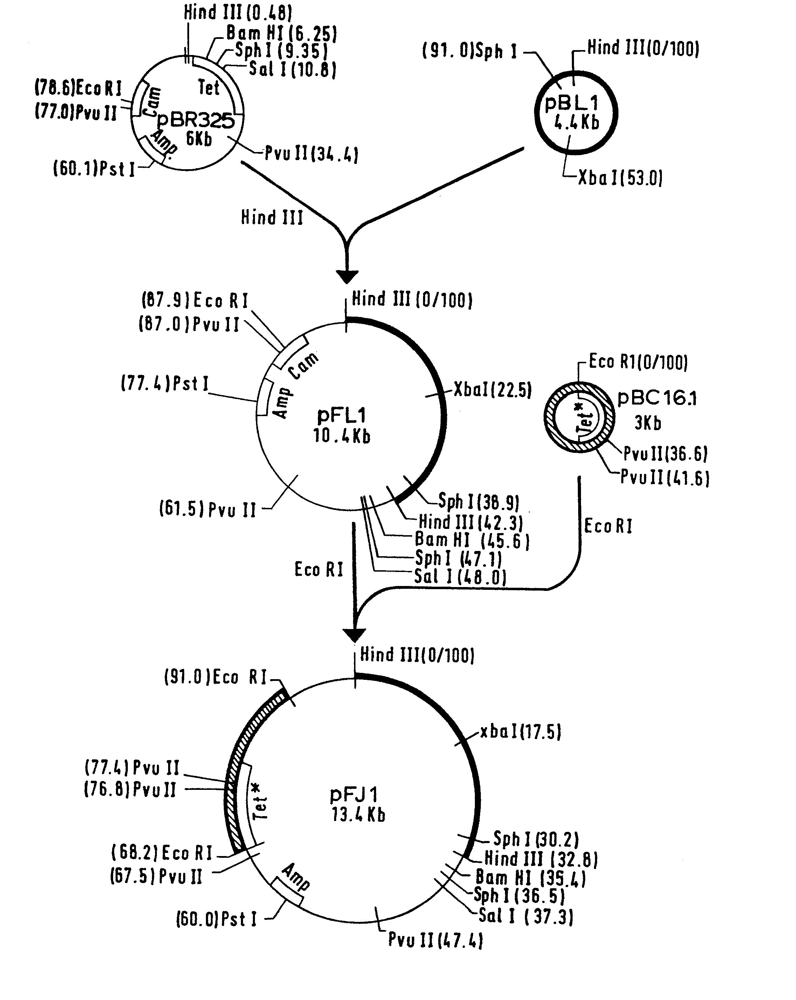

Plasmid pBL1 was treated with various restriction endonucleases under suitable conditions. The samples were incubated and subjected to agarose (0.7%) gel electrophoresis. After the electrophoresis the gel was strained with ethidium bromide and photographed under ultraviolet light. The restriction map of pBL1 shown in figure 1 was constructed by double and/or triple digestions. The restriction cleavage sites of pBL1 are also listed in the following table:

| restriction enzyme | cleavage sites |
|---|---|
| BamH I | 0 |
| Bcl I | 1 |
| Bgl II | 0 |
| Hind III | 1 |
| Kpn I | 0 |
| Pvu II | 0 |
| Sph I | 1 |
| Xba I | 1 |

(c) Construction of vector pFL1

The DNA of plasmids pBL1 and pBR325 were digested with the restriction enzyme Hind III at their respective single restriction sites. The enzyme was inactivated by incubation at 65°C for 15 minutes and the DNAs were ligated by T4 DNA ligase in the appropriate buffer for 18 hours at 12°C.

The mixture was used to transform <u>Escherichia coli</u> competent cells. Transformants were selected by plating on media containing chloramphenicol or ampicillin. Clones containing hybrid molecules were selected by their inability to grow on tetracycline replica plates, as the gene coding for this resistance in pBR325 was inactivated by insertion. Plasmid DNA was extracted from these clones. The molecular weight and restriction pattern were in agreement with the predicted parameters for a hybrid molecule. One of these clones was selected and the plasmid named pFL1. Digestion with several restriction enzymes showed that plasmid pFL1 is a composite plasmid of pBR325 and pBL1 combined at their Hind III sites, see the drawing.

Plasmid pFL1 was found to be able to transform protoplasts of coryneform bacteria expressing chloramphenicol resistance.

(d) Construction of Vector pFJ1

To make this plasmid pFL1 functional in <u>Bacillus subtilis,</u> plasmid pBC16.1 conferring tetracycline resistance (Kreft, J. et al. Molec. gen. Genet. <u>162</u> 59 (1978) was isolated in a conventional manner (Tanaka, T. et al. J. Backeriol. <u>129</u> 1487 (1977), digested with EcoR I at its unique site and ligated by T4 DNA ligase in the appropriate buffer for 18 hours at 12°C with pFL1 linearized with the same EcoR I enzyme.

The mixture was used to transform <u>Escherichia coli</u> competent cells. Transformants were selected on media containing ampicillin. Clones containing hybrid molecules were selected by their ability to grow on tetracyline and their inability to grow on chloroamphenicol replica plates: it is known that the tetracycline resistance is expressed in <u>Escherichia coli</u> (Kreft, J. et al. Molec. Gen. Genet. <u>162</u> 59 (1978). One of these clones was selected and the plasmid named pFJ1.

Plasmid pFJ1 was purified. Digestion with several restriction enzymes showed that it is a composite plasmid of pF1 and pBC16.1 with a molecular size of about 13 kilobases and the following cleavage sites for restriction endonucleases:

| restriction enzyme | cleavage sites |
|---|---|
| BamH I | 1 |
| Bcl I | 2 |
| Bgl II | 0 |
| Eco RI | 2 |
| Hind III | 2 |
| Kpn I | 0 |
| Pst I | 1 |
| Pvu II | 4 |
| Sph I | 2 |
| Xba I | 1 |

The plasmid pFJ1 was shown to carry genes for resistance to tetracycline and ampicillin.

(e) Shuttle characteristics of plasmid pFJ1

Plasmid pFJ1 was introduced by treatment of protoplasts with polyethylene glycol (PEG) in Bacillus subtilis and in Brevibacterium or Corynebacterium strains with selection of transformants in regeneration media containing tetracycline at 10 or 5 µg/ml respectively.

Plasmid pFJ1 is able to express ampicillin resistance in _Escherichia_ _coli_ and tetracycline resistance in _Escherichia_ _coli,_ _Bacillus_ _subtilis_ and _Brevibacterium_ or _Corynebacterium_ strains. The following table shows the level of resistance to tetracycline, evaluated as minimumum inhibitory concentration (MIC), due to the gene carried by the plasmid pFJ1 in _Escherichia_ _coli,_ _Bacillus_ _subtilis,_ _Corynebacterium_ _glutamicum_ and _Brevibacterium_ _lactofermentum._

| Microorganism | MIC (µg/ml) |
|---|---|
| _Escherichia_ _coli_ K12 DH1 | <2 |
| _Escherichia_ _coli_ K12 DH1/pFJ1 | 16 |
| _Bacillus_ _subtilis_ 168 1A423 | <2 |
| _Bacillus_ _subtilis_ 168 1A423/pFJ1 | 64 |
| _Brevibacterium_ _lactofermentum_ ATCC 13869 | <2 |
| _Brevibacterium_ _lactofermentum_ ATCC 13869/pFJ1 | 32 |

CLAIMS:

1. A plasmid capable of autonomous replication in microorganisms belonging to the genus Corynebacterium or the genus Brevibacterium and also in the species Bacillus subtilis and Escherichia coli.

2. A plasmid according to claim 1 which includes a selectable marker, preferably for antibiotic resistance.

3. A plasmid according to claim 1 or 2 which includes at least one uniquely cleavable restriction site.

4. A plasmid according to any of claims 1 to 3 when constructed from plasmid DNA sequences.

5. Plasmid pFJ1 contained in Bacillus subtilis NCIB 12020 or Brevibacterium lactofermentum NCIB 12021 or Escherichia coli NCIB 12023.

6. Plasmid pFJ1 characterized by a molecular size of about 13 kilobases, carrying genes for resistance to tetracycline and amplicillin, and containing the following cleavage sites for restriction endonucleases:

| restriction enzyme | cleavage sites |
|---|---|
| BamH I | 1 |
| Bcl I | 2 |
| Bgl II | 0 |
| Eco RI | 2 |
| Hind III | 2 |
| Kpn I | 0 |
| Pst I | 1 |
| Pvu II | 4 |
| Sph I | 2 |
| Xba I | 1 |

7. A plasmid showing substantial homology with the plasmid pFJ1 of claim 5 or 6.

8. Any plasmid useful as a vector for a microorganism of the genus Corynebacterium or Brevibacterium and derived from a plasmid according to any of the preceding claims by linearizing and ligating the plasmid according to any preceding claim and another DNA sequence.

9. A plasmid vector according to claim 8 wherein the DNA sequence is relevant to production of a metabolite such as an amino acid.

10. A process for producing a shuttle recombinant vector plasmid autonomously replicable in different microorganisms, characterized in that DNA sequences are spliced to give a plasmid autonomously replicable in microorganisms of the genus Corynebacterium or Brevibacterium and in microorganisms of the species Escherichia coli and Bacillus subtilis, the plasmid preferably including a gene capable of affecting biosynthesis of amino acids or other useful metabolites.